(19) Europäisches Patentamt
European Patent Office
Office européen des brevets

(11) Publication number: **0 114 333 B1**

(12) **EUROPEAN PATENT SPECIFICATION**

(45) Date of publication of patent specification: **29.08.90**

(51) Int. Cl.[5]: **A 61 K 37/02**, A 61 K 31/40, C 07 C 237/04

(21) Application number: **83112757.6**

(22) Date of filing: **19.12.83**

(54) **Pharmaceutical composition.**

(30) Priority: **27.12.82 US 453257**

(43) Date of publication of application: **01.08.84 Bulletin 84/31**

(45) Publication of the grant of the patent: **29.08.90 Bulletin 90/35**

(84) Designated Contracting States: **AT BE CH DE FR GB IT LI NL SE**

(56) References cited:
**EP-A-0 050 800**
**EP-A-0 099 239**
**US-A-4 046 889**
**CHEMICAL ABSTRACTS, vol. 88, no. 7, 1978, page 313, no. 47977f, Columbus, Ohio, US; R. IGIC et al.; "Activity of renin and angiotensin I converting enzymes in the retina and ciliary body" & LIJEC. VJESN. 1977, 99(8), 482-4**
**CHEMICAL ABSTRACTS, vol. 92, no. 25, 1980, page 368, no. 212468h, Columbus, Ohio, US; R. IGIC et al.: "Angiotensin I converting enzyme (kininase II) in ocular tissues" & EXP. EYE RES. 1980, 30(3), 299-303**

(73) Proprietor: **SCHERING CORPORATION**
**2000 Galloping Hill Road**
**Kenilworth New Jersey 07033 (US)**

(72) Inventor: **Watkins, Robert Wayne**
**154 Davey Street**
**Bloomfield New Jersey 07003 (US)**

(74) Representative: **von Kreisler, Alek, Dipl.-Chem et al**
**Patentanwälte Dr.-Ing. Schönwald Dr.-Ing. Eishold; Dr. Fues Dipl.-Chem. von Kreisler; Dipl.Chem. Keller Dipl.-Ing. Selting; Dr. Werner Deichmannhaus am Hauptbahnhof**
**D-5000 Köln 1 (DE)**

(56) References cited:
**CHEMICAL ABSTRACTS, vol. 95, no. 13, 1981, page 421, no. 112364q, Columbus, Ohio, US; J. VITA et al.: "Angiotensin-converting enzyme activity in ocular fluids" & INVEST. OPHTHALMOL. VISUAL SCI. 1981, 20(2), 255-7**

**EP 0 114 333 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Courier Press, Leamington Spa, England.

(56) References cited:
CHEMICAL ABSTRACTS, vol. 97, no. 13, 1982, page 122, no. 104746s, Columbus, Ohio, US; R. IGIC: "Activity of angiotensin I-converting enzyme (kininase II) in retina and other ocular tissues" & AGENTS ACTIONS SUPPL. 1982, AAS 9(Recent Prog. Kinins), 435-40

CHEMICAL ABSTRACTS, vol. 99, no. 21, 1983, page 421, no. 173430n, Columbus, Ohio, US; H.M. NEELS et al.: "Angiotensin I-converting enzyme activity in rabbit corneal endothelial cells" & OPHTHALMOLOGICA 1983, 187(2), 129-32

CHEMICAL ABSTRACTS, vol. 95, no. 21, 1981, page 453, no. 185021k, Columbus, Ohio, US; Y. NIITU et al.: "Serum angiotensin converting enzyme (ACE) activity in sarcoidosis in children and adults" & JPN. MED. RES. FOUND. PUBL. 1981, 13(Sarcoidosis), 211-22

## Description

Glaucoma is an ocular disease complex associated with an elevated pressure within the eye (i.e., intraocular pressure, IOP). As a result of the elevated IOP, damage to the optic nerve head resulting in irreversible loss of visual function may ensue. Untreated, this condition may eventually lead to blindness.

Ocular hypertension, i.e., the condition of elevated intraocular pressure without optic nerve head damage or characteristic glaucomatous visual field loss, is now believed by the majority of ophthalmologists to represent the earliest phase in the onset of glaucoma.

A number of the drugs presently employed to treat glaucoma are not entirely satisfactory, particularly in the earliest course of the disease when the side effects they produce are often worse than the symptoms of the disease.

Epinephrine used as a topical solution, must be utilized cautiously in patients with high blood pressure, diabetes, hyperthyroidism and cerebral artereosclerosis due to the possibility of systemic action.

Timolol, a clinically utilized, topically applied agent for lowering intraocular pressure, must be used with caution in patients in whom beta-adrenergic blockade may be undesirable. Systemic absorption of topically administered timolol and systemic beta-blockade are responsible for the contraindication of timolol therapy for glaucoma in patients with compromised pulmonary function.

Pilocarpine, a topical drug, although considered systemically harmless and quite effective, may cause considerable local difficulties. Pupil constriction may cause the eye to lose its ability to adapt from light to dark. Accommodation may become stimulated so that the patient's refraction is sometimes incorrect and vision becomes blurred. The drug itself may cause a local vasodilation and red eyes. Irritation is common.

Carbonic anhydrase inhibitors have been used systemically but they have a number of disadvantages. While effective in lowering intraocular pressure, they often cause a numbness and tingling, gastrointestinal upsets and, frequently, depression, lethargy, a loss of appetite, and general malaise. EP—A—81400326.5 attempts to overcome these difficulties by the topical administration of an alkali metal salt of a carbonic anhydrase inhibitor.

EP—A—0 050 800 discloses the formulae of pharmaceutical compositions which are not directed for use as topical ophthalmologically acceptable compositions useful for reducing and controlling the elevated intraocular pressure associated with glaucoma. It does not teach the specific topical compositions which are required for intraocular administration.

The present invention provides a pharmaceutical composition for reducing and controlling the elevated intraocular pressure associated with glaucoma.

The invention sought to be patented in its pharmaceutical composition aspect is a topical ophthalmologically acceptable composition useful for reducing and controlling the elevated intraocular pressure associated with glaucoma which comprises an intraocular pressure reducing effective amount of a pharmaceutically acceptable angiotensin converting enzyme (ACE) inhibitor in combination with an ophthalmologically acceptable carrier for topical use. The composition may contain one or more additional therapeutic agents.

The present invention relates to the use of a composition comprising

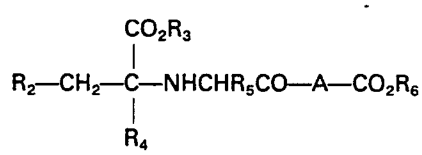 I

wherein A is

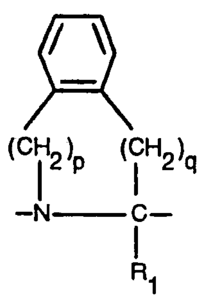 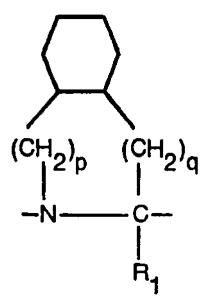 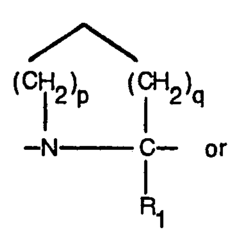 or 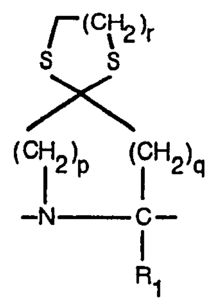

W X Y Z

$R_2$ is alkyl having from 1 to 6 carbon atoms, benzyl, benzylthio, benzyloxy, phenylthio or phenoxy;
$R_1$, $R_3$, $R_4$, and $R_6$ are the same or different and are hydrogen or alkyl having from 1 to 6 carbon atoms;
$R_5$ is hydrogen, alkyl having from 1 to 6 carbon atoms or amino alkyl having from 1 to 6 carbon atoms;
p is 0, 1 or 2;

q is 0, 1 or 2, provided that the sum of p and q is 1 or 2 and that p is not 0 in formula Z and provided that q is not 1 in formula Y when p is 1, A is Y, $R_1$, $R_4$ and $R_6$ are hydrogen, $R_2$ is benzyl, $R_3$ is ethyl, and $R_5$ is methyl;

r is 1 or 2;

and the pharmaceutically acceptable salts thereof in combination with an ophthalmologically acceptable carrier for topical use for the manufacture of a medicament for the topical treatment of glaucoma by reducing and controlling the elevated intraocular pressure associated with glaucoma.

Preferably in formula I $R_1$ and/or $R_4$ and/or $R_6$ is hydrogen and/or $R_2$ is benzyl and/or $R_5$ is methyl and/or p and/or q and/or r are 1. Preferably A is the group X, Y or Z. Preferably the compounds are of the S,S,S,-configuration.

Preferred compositions of the invention comprise ACE inhibitors having the following structural formulae:

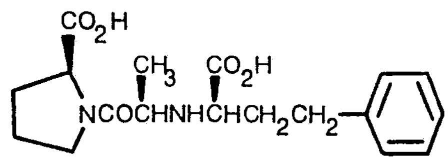
, II

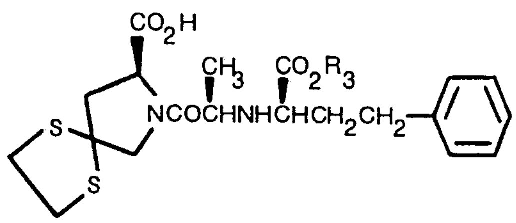
, or III

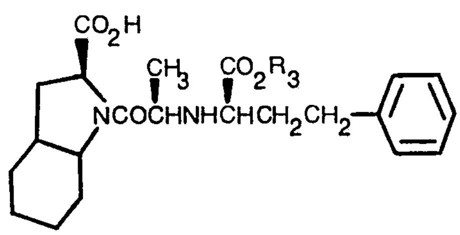
. IV

In the above formulae the heavy line (▬) utilized at the chiral centers means that the substituent so bonded is projected above the plane of the paper. The configuration at these chiral centers is denoted as "S". The substituent $R_3$ may be hydrogen or alkyl having from 1 to 6 carbon atoms. $R_3$ is preferably hydrogen or ethyl.

The invention sought to be patented in its pharmaceutical method aspect is a method for reducing and controlling the elevated intraocular pressure associated with glaucoma in a human which method comprises administering to said human an effective amount of the above-defined pharmaceutical composition.

The compounds utilized in the topical ophthalmologically acceptable pharmaceutical compositions and methods of the invention are known in the art as angiotensin converting enzyme (ACE) inhibitors. Angiotensin II, a pressor substance, is produced in vivo by the action of angiotensin converting enzyme (ACE) on angiotensin I. Compounds capable of inhibiting the action of ACE are clinically useful for controlling the blood pressure of humans suffering from hypertension. Other ACE inhibitors are known in the art, and may have a variety of structures. See, for example, An. Rev. Biochem., *51,* 283(1982) and references cited therein. It can be concluded from test results that any ACE inhibitor will possess the novel utility described herein; however, for purposes of the present invention the preferred ACE inhibitors are compounds which are capable of inhibiting the action of ACE by at least 50% at a concentration of 1 μM or less when tested by the following standard method:

ACE activity is determined by spectrophotometric assay of the product of the hydrolysis of the synthetic substrate, hippuryl histidyl leucine (HHL), as described by Cushman and Cheung, Biochem. Pharmacol., *20,* 1637(1971). The ACE used is prepared in a manner similar to that of Cheung and Cushman, Biochem. Biophys. Acta., *293,* 451(1973). Incubation for ACE assays is carried out at 37°C. Each 0.25 ml assay mixture contains the following components: 100 mM of potassium phosphate buffer containing 300 mM sodium chloride, 5 mM HHL and 1.87 mU enzyme at pH 8.3 and various concentrations of inhibitors. The enzyme reaction is terminated after 60 minutes by the addition of 0.25 ml of 1 N HCl. Inhibitors are

dissolved in appropriate solvents. Hippuric acid solution for a standard curve is prepared in a similar manner.

Each experiment involves replicate incubations for each condition to be studied. $IC_{50}$ values (the concentration required for the 50% inhibition of ACE activity) are derived from calculated regression lines. Each experiment utilizes multiple concentrations of inhibitor.

Many ACE inhibitors are known in the art and may be prepared by known methods or by variations thereof. For example, the compounds having structural formulae II and IV may be prepared as described in EP—A—81108348.4.

The compositions of this invention are considered to be no more toxic than compositions containing the ACE inhibitors for controlling hypertension.

When topically administered to the eye, the compounds of the invention reduce intraocular pressure (IOP) and were tested by the following procedure:

Male New Zealand white rabbits having a normal IOP are conditioned to the laboratory setting for at least one 4 hr period before being used to study drug effects. A Makay-Marg applanation tonometer is used to measure IOP. Readings, in mm Hg, are taken in triplicate and the average is recorded.

Rabbits are restrained in a cloth sack 2 min. prior to IOP determination. The left lower eyelid is retracted to form a pouch and 1 drop of a local anesthetic is irrigated over the cornea. The lower eyelid is then held closed over the eye for about 1 min. Corneal anesthesia is repeated before each set of IOP determinations. Readings are taken just before dosing with drug (0 time) and at hourly intervals thereafter. Drugs are administered in a 50 µl volume in the same manner as the anesthetic.

Summary of test results:
observation time: 4 hours
pretreatment intraocular pressure: 19.6—21.4 mmHg

| compound | concentration % | maximum decrease [mmHg] |
|---|---|---|
| Timolol | 0.25 | −1.7 ± 0.6 |
| | 0.5 | −2.4 ± 0.8 |
| | 1.0 | −4.4 ± 0.4 |
| | 2.0 | −3.6 ± 0.7 |
| I | 0.1 | −3.7 ± 0.9 |
| | 0.25 | −2.1 ± 0.7 |
| | 0.5 | −3.4 ± 1.1 |
| | 1.0 | −3.2 ± 0.7 |
| II | 0.25 | −3.5 ± 0.6 |
| | 0.5 | −1.2 ± 0.7 |
| | 1.0 | −2.7 ± 1.1 |

I: N-[1-(S)-Carboxy-3-phenylpropyl]-(S)-alanyl-(S)-proline
II: 1-{N-[1(S)-Carboxy-3-phenylpropyl]-(S)-alanyl}-cis,syn-octahydroindole-2(S)-carboxylic acid

The active compounds of the invention (ACE inhibitors) are administered in the form of ophthalmic pharmaceutical compositions adapted for topical administration to the eye; such as solutions, suspensions, ointments and solid inserts. Formulations of these compounds may contain from 0.01 to 5% and especially 0.25% to 2% of medicament. Other concentrations may be employed provided the dose is effective in lowering intraocular pressure. As a unit dosage form, between 0.01 to 2.5 mg., preferably 0.05 to 2.5 mg., and especially 0.1 to 1.0 mg. of the active compound is applied to the human eye, generally on a daily basis. Individual dosage requirements are variable; however, and must be administered on the basis of the severity of the disease and the response of the patient.

To prepare suitable dosage forms, the active compounds may be conveniently admixed with a non-toxic pharmaceutically acceptable carrier suitable for topical ophthalmolgic administration. Typical of such pharmaceutically acceptable carriers are, for example, water, mixtures of water and watermiscible solvents

such as lower alkanols or vegetable oils, petroleum based jelly, and including also from 0.5 to 5% by weight of hydroxyethyl cellulose, ethyl oleate, carboxymethyl cellulose, polyvinylpyrrolidone, and other water soluble ophthalmologically acceptable non-toxic polymers, for example, cellulose derivatives such as methyl cellulose, alkali metal carboxymethyl cellulose, hydroxyethyl cellulose, hydroxypropyl cellulose, hydroxypropylmethyl cellulose; acrylates such as polyacrylic acids salts, ethylacrylates; polyacrylamides; natural products such as gelatin, alginates, pectins, tragacanth, karaya, chondrus, agar, acacia; the starch derivatives such as starch acetate, hydroxyethyl starch ethers, hydroxypropyl starch, as well as other synthetic derivatives such as polyvinyl alcohol, polyvinyl pyrrolidone, polyvinyl methyl ether, polyethylene oxide, neutralized carbopol and xanthan gum and mixtures of these polymers. The pharmaceutical preparation may also contain non-toxic auxiliary substances such as emulsifying, preserving, wetting, bodying agents and the like, as for example, polyethylene glycols 200, 300, 400 and 600, carbowaxes 1,000, 1,500, 4,000, 6,000 and 10,000, antibacterial components such as quaternary ammonium compounds; phenylmercuric salts known to have cold sterilizing properties and which are non-injurious in use; thimerosal, methyl and propyl paraben, benzyl alcohol, phenyl ethanol; buffering ingredients such as alkali metal chloride, borate, acetate, gluconate buffers; antioxidants such as sodium metabisulfite, butylated hydroxyanisole (BHA), butylated hydroxytoluene (BHT) and the like; and other conventional ingredients such as sorbitan monolaurate, triethylolamine, oleate, polyoxyethylene sorbitan monopalmitylate, dioctyl alkali metal sulfosuccinate, monothioglycerol, ethylenediamine tetracetic acid and the like.

Additionally, suitable ophthalmic vehicles can be used as carrier media for the present purpose including conventional phosphate buffer vehicle systems, isotonic boric acid vehicles, isotonic alkali chloride vehicles, tris and the like.

The pharmaceutical preparation may also be in the form of a solid insert. For example, one may use a solid water soluble polymer as the carrier for the medicament. Inserts that are known in the art that are suitable for this use include those described in British Patent 15611, and in United States Patents 3,993,071; 3,986,510; 3,868,445; and 3,867,510. Solid water insoluble inserts, such as those prepared from ethylene vinyl acetate copolymer, may also be utilized.

The compositions of the invention may include additional therapeutic agents in addition to the ACE inhibitor. For example antibiotics, anesthetics as well as other IOP lowering agents may be present.

In the following formulations examples A and B stand for the active ingredients:

A: 1-{N-[1(S)-Ethoxycarbonyl-3-phenylpropyl]-(S)-alanyl}-cis,syn-octahydroindole-2(S)-carboxylic acid

B: 7-{N-[1(S)-Ethoxycarbonyl-3-phenylpropyl]-(S)-alanyl}-1,4-dithia-7-azaspiro[4.4]nonane-8(S)-carboxylic acid

* stands for the concentration of the active ingredient which is as follows:

| | active ingredient A | active ingredient B |
|---|---|---|
| Example 1 | 10 | 8 [mg/ml] |
| Example 2 | 12 | 18 [mg/ml] |
| Example 3 | 20 | 15 [mg/g] |
| Example 4 | 15 | 25 [mg/g] |
| Example 5 | 5 | [mg/ml] |

The formulations are prepared by standard procedures.

*Ophthalmic Solutions:*

| Example 1 | mg/ml |
|---|---|
| active ingredient A or B | * |
| Polyvinyl Alcohol | 20.0 |
| Sodium phosphate Dibasic | 1.2 |
| Sodium phosphate Monobasic | 0.64 |
| Edetate Disodium | 0.1 |
| Sodium Chloride | 6.0 |
| Benzalkonium Chloride | 0.1 |
| Purified Distilled Water QS. A.D. | 1.0 ml |

| Example 2 | mg/ml |
|---|---|
| active ingredient A or B | * |
| Hydroxypropyl Methylcellulose | 5.0 |
| Boric acid | 10.0 |
| Benzalkonium Chloride | 0.1 |
| Sodium Borate | 0.7 |
| Edetate Disodium | 0.1 |
| Sodium Chloride | 3.0 |
| Purified Distilled Water QS.A.D | 1.0 ml |

Ophthalmic Ointment:

| Example 3 | Mg/g. |
|---|---|
| active ingredient A or B | * |
| Purified Distilled Water | 0.1 ml |
| Methyl Paraben | 0.8 |
| Propyl Paraben | 0.1 |
| Hydrophilic Petrolatum QS. A.D. | 1.0 g |

| Example 4 | Mg/g. |
|---|---|
| active ingredient A or B | * |
| Chlorobutanol | 5 |
| Anhydous lanolin | 10 |
| Mineral Oil | 10 |
| White Petroleum QS. A.D. | 1.0 g |

Ophthalmic gel:

| Example 5 | Mg/g. |
|---|---|
| active ingredient A or B | * |
| Hydroxypropyl Methylcellulose | 40.0 |
| Boric Acid | 10.0 |
| Benzalkonium Chloride | 0.1 |
| Sodium Borate | 0.7 |
| Edetate Disodium | 0.1 |
| Purified Distilled Water QS. A.D. | 1.0 ml |

## Claims

1. The use of a composition comprising

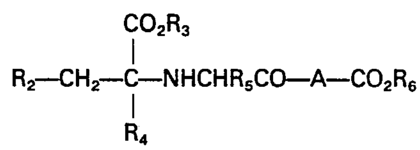

I

wherein A is

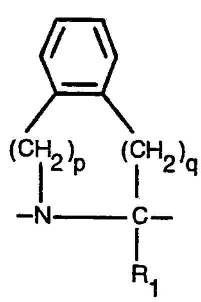
W

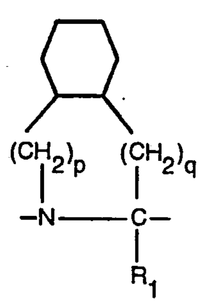
X

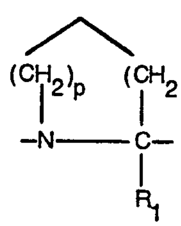
Y

or

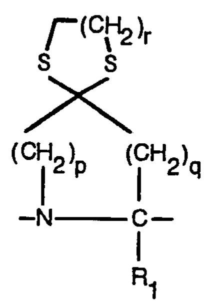
Z

$R_2$ is alkyl having from 1 to 6 carbon atoms, benzyl, benzylthio, benzyloxy, phenylthio or phenoxy; $R_1$, $R_3$, $R_4$, and $R_6$ are the same or different and are hydrogen or alkyl having from 1 to 6 carbon atoms; $R_5$ is hydrogen, alkyl having from 1 to 6 carbon atoms or amino alkyl having from 1 to 6 carbon atoms; p is 0, 1 or 2;

q is 0, 1 or 2, provided that the sum of p and q is 1 or 2 and that p is not 0 in formula Z and provided that q is not 1 in formula Y when p is 1, A is Y, $R_1$, $R_4$ and $R_6$ are hydrogen, $R_2$ is benzyl, $R_3$ is ethyl, and $R_5$ is methyl;

r is 1 or 2;

and the pharmaceutically acceptable salts thereof in combination with an ophthalmologically acceptable carrier for topical use for the manufacture of a medicament for the topical treatment of glaucoma by reducing and controlling the elevated intraocular pressure associated with glaucoma.

2. The use of a composition of claim 1, wherein in formula I $R_1$ and/or $R_4$ and/or $R_6$ is hydrogen and/or $R_2$ is benzyl and/or $R_5$ is methyl.

3. The use of a composition of claim 1 or 2, wherein in formula I p and/or q and/or r is 1.

4. The use of a composition of any one of claims 1 to 3 wherein in formula I A is the group X, Y or Z.

5. The use of a composition of any one of claims 1 to 4, wherein said ACE inhibitor is

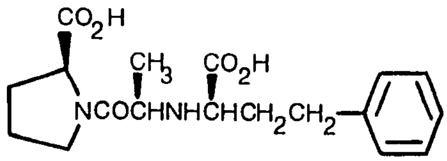
, II

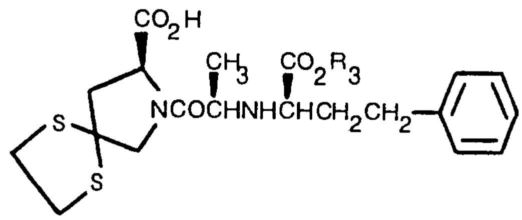
, or III

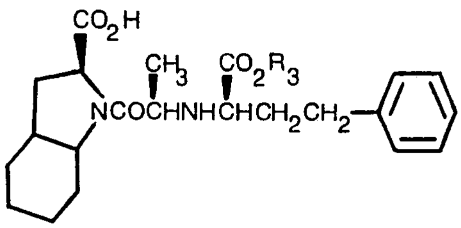
. IV

6. The use of a composition according to any one of claims 1 to 5, wherein $R_3$ is hydrogen or ethyl.

7. The use of a composition as defined in any one of claims 1 to 6 in the form of a dosage unit for the manufacture of a medicament for the topical treatment of glaucoma.

8. Use of a composition for the manufacture of a medicament for the topical treatment of glaucoma by reducing and controlling the elevated intraocular pressure associated with glaucoma characterized by admixing an intraocular pressure reducing effective amount of a pharmaceutically acceptable angiotesin converting enzyme inhibitor with an ophthalmologically acceptable carrier for topical use, said ACE inhibitor being a compound having structural formula

$$R_2\text{—}CH_2\text{—}\overset{\overset{\displaystyle CO_2R_3}{|}}{\underset{\underset{\displaystyle R_4}{|}}{C}}\text{—}NHCHR_5CO\text{—}A\text{—}CO_2R_6 \qquad I$$

wherein A is

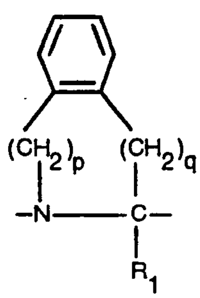
W

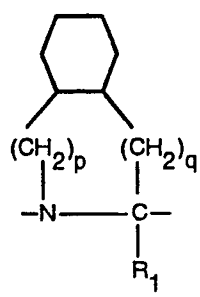
X

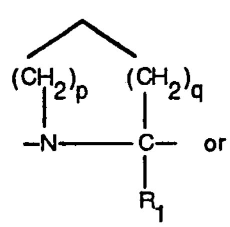
or

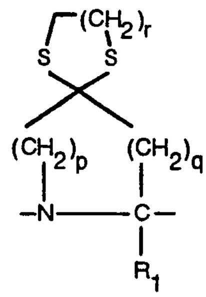
Z

$R_2$ is alkyl having from 1 to 6 carbon atoms, benzyl, benzylthio, benzyloxy, phenylthio or phenoxy;
$R_1$, $R_3$, $R_4$, and $R_6$ are the same or different and are hydrogen or alkyl having from 1 to 6 carbon atoms;
$R_5$ is hydrogen, alkyl having from 1 to 6 carbon atoms or amino alkyl having from 1 to 6 carbon atoms;
p is 0, 1 or 2;
q is 0, 1 or 2, provided that the sum of p and q is 1 or 2 and that p is not 0 in formula Z and provided that q is not 1 in formula Y when p is 1, A is Y, $R_1$, $R_4$ and $R_6$ are hydrogen, $R_2$ is benzyl, $R_3$ is ethyl, and $R_5$ is methyl;
r is 1 or 2;
and the pharmaceutically acceptable salts thereof.

9. Use of a composition for the manufacture of a medicament for the topical treatment of glaucoma by reducing and controlling the elevated intraocular pressure associated with galucoma characterized by admixing an intraocular pressure reducing effective amount of a pharmaceutically acceptable angiotensin converting enzyme inhibitor with an ophthamologically acceptable carrier for topical use, said ACE inhibitor being a compound having structural formula

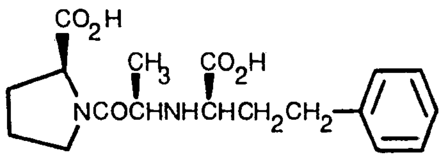 , II

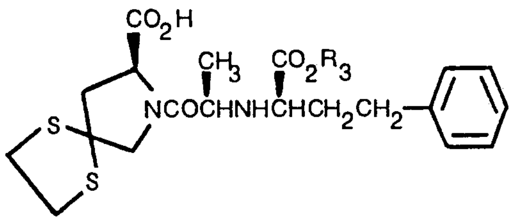 , or III

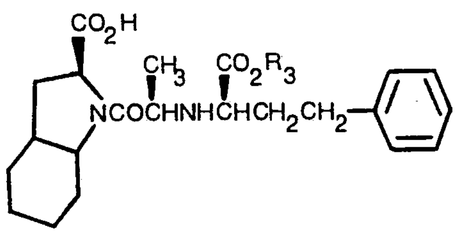 . IV

wherein $R_3$ is hydrogen or ethyl.

**Patentansprüche**

1. Verwendung einer Zusammensetzung umfassend

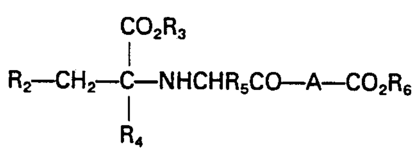 I

worin A

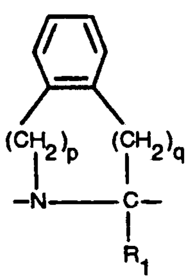
W

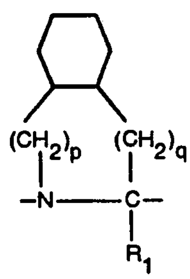
X

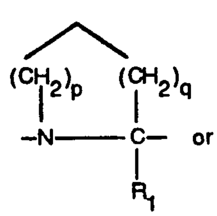
Y

or

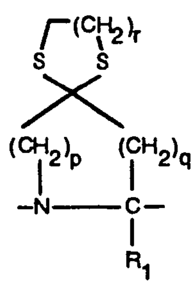
Z

ist;

$R_2$ Alkyl mit 1 bis 6 C-Atomen, Benzyl, Benzylthio, Benzyloxy, Phenylthio oder Phenoxy ist;

$R_1$, $R_3$, $R_4$ und $R_6$ gleich oder verschieden sind und Wasserstoff oder Alkyl mit 1 bis 6 C-Atomen sind;

$R_5$ Wasserstoff, Alkyl mit 1 bis 6 C-Atomen oder Aminoalkyl mit 1 bis 6 C-Atomen ist;

p 0, 1 oder 2 ist;

q 0, 1 oder 2 ist, vorausgesetzt daß die Summe von p und q 1 oder 2 ist und daß p in der Formel Z nicht 0 ist und vorausgesetzt daß q in der Formel Y nicht 1 ist, wenn p 1 ist, A Y ist, $R_1$, $R_4$ und $R_6$ Wasserstoff sind, $R_2$ Benzyl ist, $R_3$ Ethyl ist und $R_5$ Methyl ist;

r 1 oder 2 ist;

und die pharmazeutisch akzeptablen Salze davon in Kombination mit einem ophthalmologisch akzeptablen Träger zur Verwendung für die Herstellung eines Medikaments für die örtliche Behandlung von Glaucoma durch Reduktion und Kontrolle des erhöhten Augeninnendrucks, der mit Glaucoma verbunden ist.

2. Verwendung einer Zusammensetzung nach Anspruch 1, wobei in der Formel I $R_1$ und/oder $R_4$ und/oder $R_6$ Wasserstoff und/oder $R_2$ Benzyl und/oder $R_5$ Methyl ist.

3. Verwendung einer Zusammensetzung nach Ansprüchen 1 oder 2, worin in der Formel I p und/oder q und/oder r 1 ist.

4. Verwendung einer Zusammensetzung nach irgendeinem der Ansprüche 1 bis 3, worin in Formel I A die Gruppe X, Y oder Z ist.

5. Verwendung einer Zusammensetzung nach irgendeinem der Ansprüche 1 bis 4, worin der ACE-Inhibitor

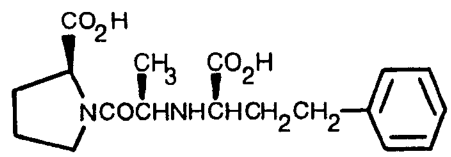 , II

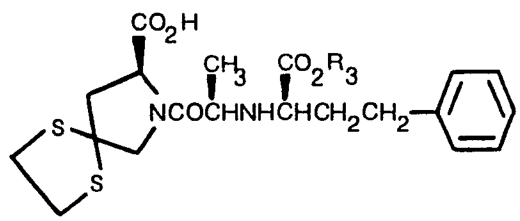 , oder III

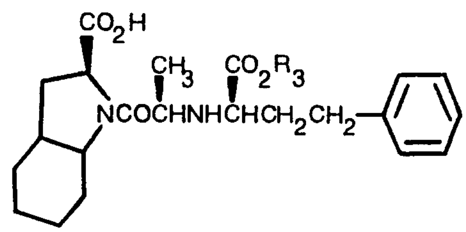 . IV

6. Verwendung einer Zusammensetzung nach irgendeinem der Ansprüche 1 bis 5, worin $R_3$ Wasserstoff oder Ethyl ist.

7. Verwendung einer Zusammensetzung wie in irgendeinem der Ansprüche 1 bis 6 definiert, in der Form einer Dosiereinheit für die Herstellung eines Medikaments für die örtliche Behandlung von Glaucoma.

8. Verwendung einer Zusammensetzung für die Herstellung eines Medikaments für die örtliche Behandlung von Glaucoma durch Reduktion und Kontrolle des erhöhten Augeninnendrucks, der mit Glaucoma verbunden ist, dadurch gekennzeichnet, daß eine wirksame, den Augeninnendruck reduzierende Menge eines pharmazeutisch akzeptablen Angiotensin-Converting-Enzym (ACE)-Inhibitors mit einem ophthalmologisch akzeptablen Träger für die örtliche Verwendung gemischt wird, wobei der ACE-Inhibitor eine Verbindung der Strukturformel

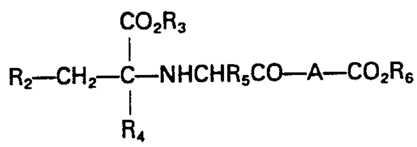 I

ist,
worin A

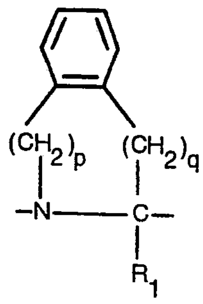 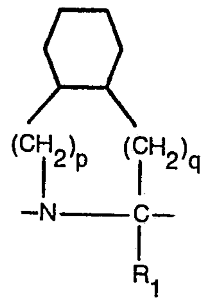 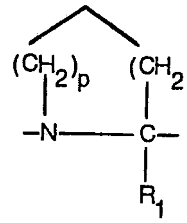 or 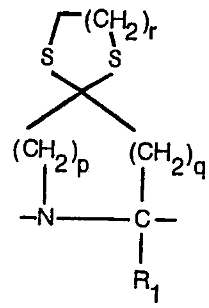

W X Y Z

ist,

$R_2$ Alkyl mit 1 bis 6 C-Atomen, Benzyl, Benzylthio, Benzyloxy, Phenylthio oder Phenoxy ist;
$R_1$, $R_3$, $R_4$ und $R_6$ gleich oder verschieden sind und Wasserstoff oder Alkyl mit 1 bis 6 C-Atomen sind;
$R_5$ Wasserstoff, Alkyl mit 1 bis 6 C-Atomen oder Aminoalkyl mit 1 bis 6 C-Atomen ist;
p 0, 1 oder 2 ist;
q 0, 1 oder 2 ist, vorausgesetzt daß die Summe von p und q 1 oder 2 ist und daß p in der Formel Z nicht 0 ist und vorausgesetzt daß q in der Formel Y nicht 1 ist, wenn p 1 ist, A Y ist, $R_1$, $R_4$ und $R_6$ Wasserstoff sind, $R_2$ Benzyl ist, $R_3$ Ethyl ist und $R_5$ Methyl ist;
r 1 oder 2 ist;
und die pharmazeutisch akzeptablen Salze davon.

9. Verwendung einer Zusammensetzung für die Herstellung eines Medikaments für die örtliche Behandlung von Glaucoma durch Reduktion und Kontrolle des erhöhten Augeninnendrucks, der mit Glaucoma verbunden ist, dadurch gekennzeichnet, daß eine wirksame, den Augeninnendruck reduzierende Menge eines pharmazeutisch akzeptablen Angiotensin-Converting-Enzym (ACE)-Inhibitors mit einem ophthalmologisch akzeptablen Träger für die örtliche Verwendung gemischt wird, wobei der ACE-Inhibitor eine Verbindung der Strukturformel

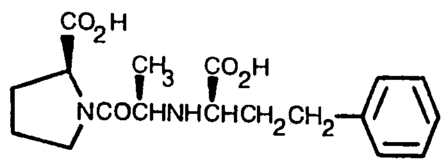 , II

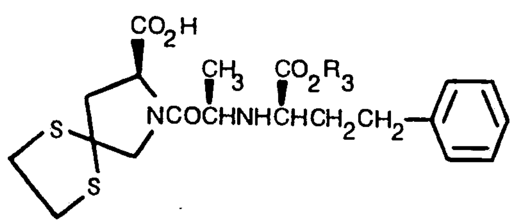 , oder III

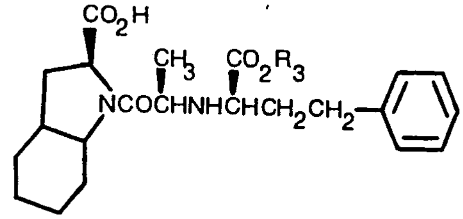 IV

ist, wobei $R_3$ Wasserstoff oder Ethyl ist.

**Revendications**

1. Utilisation d'une composition comprenant:

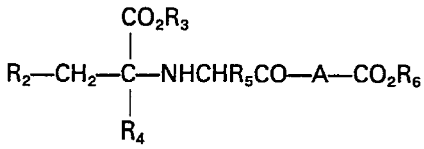

I

où A est:

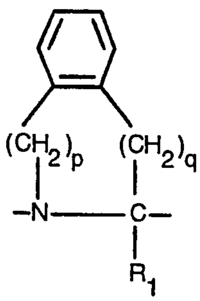

W

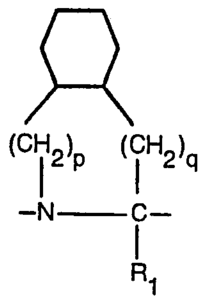

X

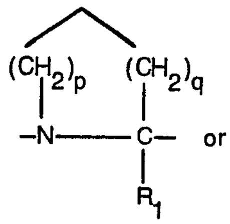

Y

or

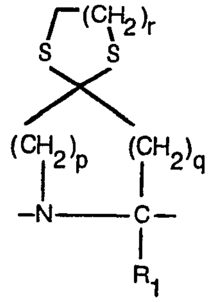

Z

$R_2$ est alkyle ayant de 1 à 6 atomes de carbone, benzyle, benzylthio, benzyloxy, phénylthio ou phénoxy;

$R_1$, $R_3$, $R_4$ et $R_6$ sont identiques ou différents et sont hydrogène ou alkyle ayant de 1 à 6 atomes de carbone;

$R_5$ est hydrogène, alkyle ayant de 1 à 6 atomes de carbone ou amino alkyle ayant de 1 à 6 atomes de carbone;

p est 0, 1 ou 2;

q est 0, 1 ou 2, à condition que la somme de p et q soit 1 ou 2 et que p ne soit pas 0 dans la formule Z et à condition que q ne soit pas 1 dans la formule Y quand p est 1, A est Y, $R_1$, $R_4$ et $R_6$ sont hydrogène, $R_2$ est benzyle, $R_3$ est éthyle et $R_5$ est méthyle;

r est 1 ou 2;

et leurs sels acceptables en pharmacie en combinaison avec un véhicule acceptable en ophtalmologie pour un usage topique pour la fabrication d'un médicament pour le traitement topique du glaucome par réduction et contrôle de la pression intra-oculaire élevée associée au glaucome.

2. Utilisation d'une composition de la revendication 1, où dans la formule I, $R_1$ et/ou $R_4$ et/ou $R_6$ est hydrogène et/ou $R_2$ est benzyle et/ou $R_5$ est méthyle.

3. Utilisation d'une composition de la revendication 1 ou 2, où dans la formule I, p et/ou q et/ou r est 1.

4. Utilisation d'une composition selon l'une quelconque des revendications 1 à 3, où dans la formule I, A est le groupe X, Y ou Z.

5. Utilisation d'une composition selon l'une quelconque des revendications 1 à 4, où ledit inhibiteur de ACE est

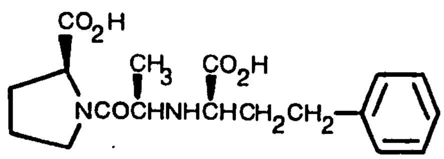

, II

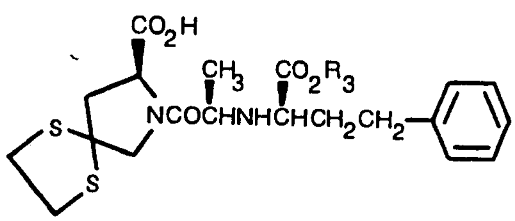

, ou III

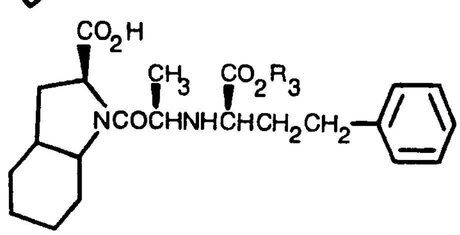

. IV

6. Utilisation d'une composition selon l'une quelconque des revendications 1 à 5, où $R_3$ est hydrogène ou éthyle.

7. Utilisation d'une composition selon l'une quelconque des revendications 1 à 6 sous la forme d'une unité de dosage pour la fabrication d'un médicament pour le traitement topique du glaucome.

8. Utilisation d'une composition pour la fabrication d'un médicament pour le traitement topique du glaucome en réduisant et en contrôlant la pression intra-oculaire élevée associée au glaucome, caractérisée par le mélange d'une quantité efficace de réduction de la pression intra-oculaire d'un inhibiteur de l'enzyme de conversion de l'angiotensine acceptable en pharmacie avec un véhicule acceptable en ophtalmologie pour un usage topique, ledit inhibiteur de ACE étant un composé ayant la formule de structure:

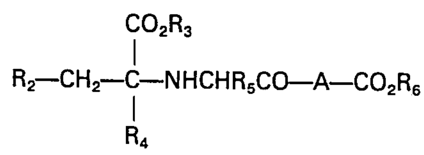

I

où A est:

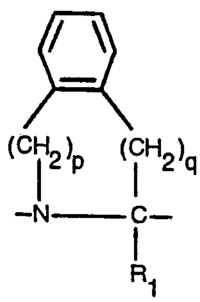

W

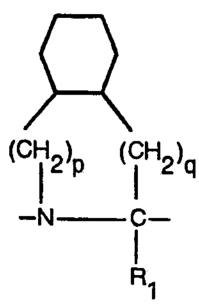

X

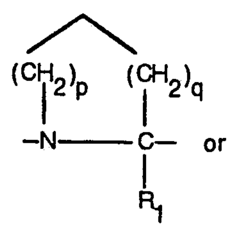

Y

or

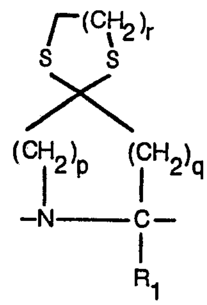

Z

$R_2$ est alkyle ayant de 1 à 6 atomes de carbone, benzyle, benzylthio, benzyloxy, phénylthio ou phénoxy;

$R_1$, $R_3$, $R_4$ et $R_6$ sont identiques ou différents et sont hydrogène ou alkyle ayant de 1 à 6 atomes de carbone;

$R_5$ est hydrogène, alkyle ayant de 1 à 6 atomes de carbone ou amino alkyle ayant de 1 à 6 atomes de carbone;

p est 0, 1 ou 2;

q est 0, 1 ou 2, à condition que la somme de p et q soit 1 ou 2 et que p ne soit pas 0 dans la formule Z et à condition que q ne soit pas 1 dans la formule Y quand p est 1, A est Y, $R_1$, $R_4$ et $R_6$ sont hydrogène, $R_2$ est benzyle, $R_3$ est éthyle et $R_5$ est méthyle;

r est 1 ou 2;

et leurs sels acceptables en pharmacie.

9. Utilisation d'une composition pour la fabrication d'un médicament pour le traitement topique du glaucome en réduisant et en contrôlant la pression intra-oculaire élevée associée au glaucome, caractérisée par le mélange d'une quantité efficace réduisant la pression intra-oculaire d'un inhibiteur de l'enzyme de conversion de l'angiotensine acceptable en pharmacie avec un véhicule acceptable en ophtalmologie pour usage topique, ledit inhibiteur de ACE étant un composé ayant la formule de structure

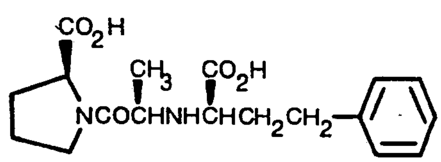

, II

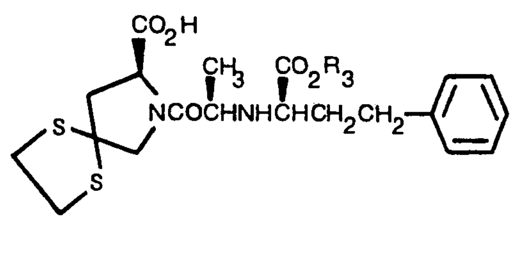

, ou III

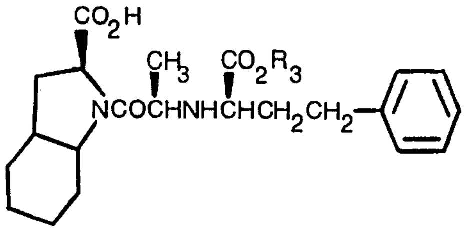 . IV

où $R_3$ est hydrogène ou éthyle.